Europäisches Patentamt

European Patent Office

Office européen des brevets

(19)

(11) Publication number: **0 401 956**
**A2**

## EUROPEAN PATENT APPLICATION

(12)

(21) Application number: 90302002.2

(22) Date of filing: 26.02.90

(51) Int. Cl.5: **A23L 1/23, A23G 3/30**

(30) Priority: **05.06.89 US 361518**

(43) Date of publication of application:
**12.12.90 Bulletin 90/50**

(84) Designated Contracting States:
**BE CH DE DK ES FR GB GR IT LI NL SE**

(71) Applicant: **WARNER-LAMBERT COMPANY**
**201 Tabor Road**
**Morris Plains New Jersey 07950(US)**

(72) Inventor: **Cherukuri, Subraman Rao**
**10 Jean Drive**
**Towaco, New Jersey 07082(US)**
Inventor: **Mansukhani, Gul**
**97 Petrus Avenue**
**Staten Island, New York 10312(US)**
Inventor: **Chau, Tommy Limkwong**
**3 Dartmouth 3A**
**Bridgewater, New Jersey 08807(US)**

(74) Representative: **Bond, Bentley George et al**
**HASELTINE LAKE & CO. Hazlitt House 28**
**Southampton Buildings Chancery Lane**
**London, WC2A 1AT(GB)**

(54) High melting point sweetener delivery system.

(57) A high melting-point sweetener delivery system useful in chewing gum, confectionary, comestibles, personal products and pharmaceutical compositions, said delivery system compromising a particulate sweetener encapsulated in a matrix consisting essentially of a blend of a fat and rosin ester. The melting point of the delivery system is about 60 to about 70°C.

EP 0 401 956 A2

## HIGH MELTING POINT SWEETENER DELIVERY SYSTEM

This invention relates to high melting point sweetener delivery systems as well as ingestible compositions containing them. In particular this invention relates to a particulate sweetener encapsulated by a matrix consisting essentially of a blend of selected rosins and fats such that the melting point of the delivery system is above 60°C and more preferably about 65 to about 70°C.

The higher melting point of the instant delivery systems allows these delivery systems to be incorporated in chewing gum and confectionery compositions which can then be processed at higher than normal temperatures. For example, it is well known that currently most chewing gum compositions containing sweetener delivery systems have to be processed at temperatures between about 40° and 43°C in order to keep the encapsulation intact. At temperatures higher than this, for example 44°-50°C, the encapsulation matrix begins to melt appreciably, thereby prematurely releasing the sweetener incorporated therein. Additionally, frictional forces during processing tend to create rupture of the encapsulating matrix, especially after the matrix is substantially softened. The result, of course, is a loss of the delayed and controlled sweetener release normally provided by the delivery system. Additionally, premature melting and breakdown of the delivery system fails to provide the required protection and separation of sensitive sweeteners from other gum components which may cause sweetener instability. Such instability may cause degradation of the sweetener per se, discoloration due to interaction with other chemical constituents in the chewing gum composition, loss of shelf-life and an overall inferior gum product.

Presently, chewing gum compositions which incorporate sweetener delivery systems are processed at the lower temperatures, e.g., 40 - 43°C to prevent the aforementioned deleterious effects. These temperatures are not, however, the best for extruding and processing the chewing gum composition into a final product. Conventional temperatures which are optimum for extruding chewing gum compositions have been found to be about 48° to about 54°C. The prior art delivery systems suffer from all of these, among other, shortcomings.

Numerous sweetener delivery systems are currently used in edible products, and particularly in chewing gum. For example, U.S. Patent 4,597,970 concerns sweetener delivery systems which contain an encapsulating matrix for sweeteners which matrix comprises lecithin, a glyceride and either a fatty acid, natural or synthetic wax or mixtures of these three latter groups. Although this delivery system is adequate for certain purposes, and discloses a composite delivery system with a melting point of about 25° to 100°C, it fails to disclose the composition of the instant invention.

U.S. Patent 4,722,845 discloses a cinnamon flavored chewing gum composition having improved color, sweetener and flavor stability in the presence of amino acid or dipeptide sweeteners comprising a gum base, a cinnamon flavor and a stable sweetener delivery system capable of effecting a controlled release of the sweetener, said delivery system comprising an admixture of:

(A) a hydrogenated or partially hydrogenated fat selected from the group consisting of soybean oil, palm oil, palm kernel oil, peanut oil, rapeseed oil, rice bran oil, cottonseed oil, sunflower oil, safflower oil and mixtures thereof;

(B) polyethylene wax having a melting point of about 106°C or greater.

(C) glyceride in the amount of about 0.5% to about 20% by weight of the total chewing gum composition; and

(D) an amino acid or dipeptide sweetener; wherein said sweetener is encapsulated by the admixture of components A through C.

This reference discloses the use of fat in amounts of about 63% to about 90% by weight of the delivery system. The polyethylene wax is present in amounts of about 1% to about 25% by weight of the delivery system and has a molecular weight range of about 500 to about 10,000.

U.S. Patent 4,087,557 discloses a chewing gum composition having an artificial sweetener dissolved or otherwise entrapped, i.e. coated or dispersed, in the ester gum component of the gum base, thereby providing a controlled release of the sweetener into the saliva. The ester gum component comprises the methyl, glycerol or pentaerythritol esters of the rosins or modified rosins, such as hydrogenated, dimerized or polymerized rosins or mixtures thereof. Examples include the pentaerythritol ester of partially hydrogenated wood rosin, glycerol ester of partially dimerized rosin, glycerol ester of wood rosin, as well as other water soluble ester gums. Tackifiers and lubricants may be added to the gum base separately, as well as softeners such as fats having a melting point of about 22°C. Such softening agents are generally disclosed as being useful in amounts of up to 15% by weight. To entrap the sweetener in the gum base, the ester gum is heated to 180°F to 240°F and the particulate is added and mixed thoroughly to form a dispersion. The mixture is removed from the kettle, cooled, ground into particles and then added to the chewing gum composition.

U.S. Patent 4,445,821 discloses a chewing gum having prolonged flavor or sweetness by means of delivering the flavor or sweetener in a two phase wax system. The inner phase is comprised of a solid solution of the flavor or sweetener in a wax having a functional group. Examples of such wax are fatty acids such as stearic acid or beeswax. The flavor or sweetener is miscible within the wax. This inner phase is then admixed with another wax phase which does not contain functional groups and in which the flavor or sweetener is not miscible. Examples of such waxes are polyethylene and paraffin wax. The wax systems are prepared by heating the inner wax to temperatures of 60-70°C to soften it, at which time the flavor and/or sweetener is added. The outer wax is heated, for example, in the case of polyethylene, to 140-150°C and then combined with the inner wax mixture, cooled by allowing to drip from a valve onto a cool plate, comminuted and added to a chewing gum or confectionery. In the case of addition to a chewing gum base, the gum base is preheated to a temperature of 60-80°C and the combined wax system is then added.

The inventive high melting-point delivery systems are designed to be used at higher processing and temperature conditions than the prior art sweetener delivery systems. Thus, the inventive delivery systems can be incorporated into chewing gum compositions which are processed at temperatures of about 48-54°C. This allows for optimum processing, extruding and manufacturing conditions of the gum, without danger of loss of sweetener due to rupture of the matrix or temperature degradation of the sweetener.

Other ingestible compositions such as confectionery, pharmaceutical or mouthwash and dentifrice compositions may also employ the inventive delivery system. The delivery systems are, however, especially designed for chewing gum compositions and allow for higher, more convenient gum processing and extruding temperatures to be employed without rupture, melting or substantially softening of the encapsulating matrix which normally results in premature loss of sweetener.

The inventive delivery systems thus comprise a particulate sweetener encapsulated by a matrix consisting essentially of a blend of rosins and fats, wherein the rosins are selected from the group consisting of hydrogenated and partially hydrogenated wood or gum rosin esters and mixtures thereof, and the fats are selected from the group consisting of hydrogenated and partially hydrogenated soybean oil, cottonseed oil and mixtures thereof. The blend must be such that the melting point is above at least about 55°C, preferably about 60 to 70°C, and most preferably about 65 to 67°C.

The melting point and melting range of the delivery systems tested in the Examples were determined using Differential Scanning Colorimetry (DSC). This technique measures the differential in heat flow between the test sample and a reference standard. The melting point is established when the test sample demonstrates a maximum amount of heat absorbed. This point is usually at or close to the phase change from a solid or semi-solid phase to a melted or liquid phase. Standard DSC equipment is used to perform this type of test. Samples of the delivery systems, generally about 6 to about 10 mg by weight, are used to obtain the melting profile. Results are converted electronically and represented graphically in Figures I and II.

Figure I is a graphic representation of a sweetener delivery system using only fat (soybean oil, Table I, Composition B) as the encapsulating matrix. The delivery system particles were not formed using spray congealing techniques due to the unduly high viscosity of the fat. Instead hand mixing of the sweetener into the fat was required. Thus, although the melting point of this delivery system is high enough to be useful in and tolerant of gum processing temperatures in the range of 48° to 54°C, it is not commercially viable since it must be mechanically admixed rather than spray congealed.

Figure II is a graphic representation of inventive composition G (Table I). The melting point range shows peaks at 61.2° and 65.7°C. This delivery system was easily spray congealed and formed at high speed, commercially feasible rates. Chewing gum compositions were made incorporating this delivery system at temperatures ranging from 48° to 54°C without damage to the encapsulation or premature rupturing or melting.

In addition to higher melting point capability, it is necessary that the delivery system be easily processed and formed such that it can be manufactured without additional cost or special manufacturing considerations. This is important both for economic commercial viability and for quality control. While a number of encapsulated compositions may be formed having melting points well above those used to process and form chewing gum compositions, not all would also possess the requirement of efficient, cost effective processing, as well as satisfying the overall requirements of compatibility with other chewing gum components and pleasing organoleptic properties. For example, as evidenced by the Examples described herein, encapsulation only with fat results in a relatively high melting point encapsulant. Yet, such an encapsulant is commercially unprocessable due to the high viscosity of the fat/sweetener mixture. As later discussed, attempts to process such an encapsulant using commercial techniques failed. Additionally, the viscosity was so high as to render dispersion of the sweetener within the fat difficult. This resulted in lumping of the sweetener rather than the uniform dispersion required to properly form the encapsulant.

One attempt known in the art to remedy the deficiencies of fat encapsulants was to add various emulsifiers. Emulsifiers such as glyceryl monostearate and lecithin are commonly used. While the addition of emulsifiers tends to facilitate the processability of the fat mixture, it also tends to lower rather than increase the melting point.

Thus, while various attempts to deliver sweeteners via encapsulating systems have been tried, none have the higher melting point range of the instant invention which allows for better retention of the sweetener during gum processing.

The particulate sweeteners may be selected from a wide group of natural and artificial sweeteners. These sweeteners include sucrose, chlorinated sucrose derivatives such as sucralose, glucose (corn syrup), dextrose, invert sugar, fructose, dihydrochalcone compounds, talin, monellin, glycyrrhizin, steviosides, saccharin acid and its various salts, cyclamic acid and it various salts, amino acid-based and dipeptide sweeteners such as aspartame and its derivatives, sugar alcohols such as sorbitol, mannitol, xylitol and the like, hydrogenated starch hydrolysate (lycasin), and 3, 6-dihydro-6-methyl-1-1,2,3-oxathiazin-4-one, 2,2-dioxide, particularly the potassium (Acesulfame-K), sodium and calcium salts thereof. Mixtures of these sweeteners are contemplated. The use of the high intensity sugarless sweeteners are preferred for incorporation into the encapsulating matrix of the inventive delivery systems.

In the instance where auxiliary sweeteners are utilized in the final ingestible product in addition to those in the delivery system per se, the present invention contemplates the inclusion of those sweeteners well known in the art, including both natural and artificial sweeteners. Thus, additional sweeteners may be chosen from the same non-limiting list as those in the delivery system.

The sweeteners in the delivery system per se, may be used in amounts of about 0.01% to about 35% by weight of the delivery system. Aspartame and saccharin (and its salts) are the preferred sweeteners and may be used in amounts of about 0.01% to about 25% and about 0.01% to 50% respectively, by weight of the delivery system. The preferred amounts of these sweeteners are about 2% to about 16%. Auxiliary sweeteners may be used in conventional amounts based on the total weight of the chewing gum compositions, as is standard in the art.

A particularly effective combination of sweeteners is aspartame, sodium saccharin and acesulfame K (potassium acesulfame). Saccharin and its salts and acesulfame salts may be employed in amounts of about 5% to about 50% by weight of the delivery system. Aspartame is used in amounts up to about 15% by weight when used in this combination. One or more of the sweeteners may be in the encapsulated form prior to incorporation in the delivery system, thus delaying the release of the sweetener even more and lengthening the perceptible sweetness and or staggering their release. Thus, the sweeteners may be incorporated such that they release sequentially.

It has also been discovered that the incorporation of the combination of two or more sweeteners used alone or as part of the delivery system improves the sweetness intensity as a result of synergism. Exemplary examples of synergistic combinations are saccharin/aspartame; saccharin/potassium acesulfame; and saccharin/aspartame/potassium acesulfame. These sweetener combinations can also be added to a final food, confectionery, drug or personal product independently of the delivery system. Thus, a food product may have sweetness imparted thereto attributable to the delivery system sweetness as well as those auxiliary sweeteners independent of the delivery system. These combinations of sweeteners have also been discovered to impart a long standing flavor effect to a food product such as chewing gum.

If the sweetener has an irregular shape, i.e. aspartame, making it difficult to adequately coat with the fat/rosin mixture, two coating steps are used. Spray congealing is first employed to orient the sweetener crystals into an agglomeration which is easier to coat. The agglomeration is then easily coated a second time to ensure coverage of crystalline corners and crevices using fluidized bed granulation techniques.

A balance must be maintained however, such that too thick a coating is not used so as to prevent proper release of the sweetener material. It has been determined that using the total exterior coating in amounts of about 200 to about 300% by weight of the delivery system maximizes the protective benefits i.e., improved stability in the presence of moisture and reactive flavors, with the controlled release benefits. Use of the exterior coating in less than amounts of about 30% by weight of the delivery system does not provide thickness sufficient for protection of the core material against moisture and flavor interactions.

With regard to the chewing gum formulation in which the novel delivery system is employed, the amount of gum base employed will vary greatly depending on various factors such as the type of base used, consistency desired and other components used to make the final product. In general, amounts of about 5% to about 50% by weight of the final chewing gum composition are acceptable for use in chewing gum compositions with preferred amounts being in the range of about 15% to about 25% by weight. The gum base may be any water-insoluble gum base well known in the art. Illustrative examples of suitable polymers in gum bases include both natural and synthetic elastomers and rubbers. For example, those polymers which are suitable in gum bases include, without limitation, substances of vegetable origin such as chicle, jelutong, gutta percha and crown gum. Synthetic elastomers such as butadiene-styrene copolymers, isobutylene-isoprene copolymers, polyethylene, polyisobutylene, polyvinylacetate and mixtures thereof, are particularly useful.

The gum base composition may contain elastomer solvents to aid in softening the elastomer component. Such solvents may comprise methyl, glycerol or pentaerythritol esters of rosins or modified rosins, such as hydrogenated, dimerized or polymerized rosins or mixtures thereof. Examples of elastomer solvents suitable for use herein include the pentaerythritol ester of partially hydrogenated wood rosin, pentaerythritol ester of partially dimerized rosin, glycerol ester of polymerized rosin, glycerol ester of tall oil rosin, glycerol ester of wood rosin and partially hydrogenated wood rosin and partially hydrogenated methyl ester of rosin, such as polymers of alpha-pinene or beta-pinene; and terpene resins including polyterpene and mixtures thereof. The solvent may be employed in an amount ranging from about 10% to about 75% and preferably about 45% to about 70% by weight of the gum base.

Flavoring agents will known to the chewing gum art may be added to the chewing gum compositions of the instant invention. Suitable flavorings include both natural and artificial flavors. Examples include mint flavors such as peppermint, spearmint, menthol; citrus flavors such as, grape, cherry, pineapple, apple, orange, raspberry and banana. Mixtures are of course contemplated. The flavorings are generally utilized in amounts that will vary depending upon the individual flavor, and may, for example, range in amounts of about 0.5% to about 3% by weight of the final chewing gum composition weight.

The colorants useful in the present invention include the pigments such as titanium dioxide, that may be incorporated in amount up to about 1% by weight, and preferably up to about 0.6% by weight. Also, the colorants may include other dyes suitable for food, drug and cosmetic applications, known as F.D & C. dyes and the like. The materials acceptable for the foregoing spectrum of use are preferably water-soluble. Illustrative examples include indigoid dye, known as F.D. & C. Blue No. 2, which is the disodium salt of 5,5′-indigotindisulfonic acid. Similarly, the dye known as F.D. & C. Green No. 1, comprises a triphenylmethane dye and is the monosodium salt of 4-[4-N-ethyl-p-sulfobenzylamino)diphenylmethylene]-[1-(N-ethyl-N-p-sulfoniumbenzyl)- 2,5-cyclohexadienimine]. A full recitation of all F.D. & C. and D. & C. and their corresponding chemical structures may be found in the Kirk-Othmer Encyclopedia of Chemical Technology, in Volume 5, at Pages 857-884.

The inventive compositions are formed by the process comprising the steps of:

1. Premixing the fat and rosin to form a blended mixture; and

2. Mixing into the mixture of Step 1. the particulate sweetener until a uniform dispersion of the sweetener is obtained; and

3. Spray congealing the total mixture to form discrete, solidified encapsulated sweetener particles.

Step 1 of the above-referenced process is carried out by fully melting the rosin in a steam jacketed kettle. Once the rosin is melted, the fat (cottonseed or soybean oil) is slowly mixed into the melted rosin and the mixture is liquified to a substantially homogeneous mass having a viscosity of about 88 to about 100 CPS. The temperature at which the rosin and fat are combined is approximately 88° to about 90° C. At this point the particulate sweetener powder is added slowly in steps to facilitate dispersion in the rosin/fat mixture without clumping. This takes anywhere from ten to twenty-five minutes to complete, at which time the mixture has cooled to about 80° C. The warm sweetener containing mixture is then fed into a heat controlled spray nozzle and spray congealed. The term "spray congealing" as used herein refers to the solidification of the atomized liquid droplets which cool and solidify upon hitting the cooler temperature of the surrounding atmosphere. The nozzle pressure is regulated to control the particle droplet size. The droplets cool and congeal once they are emitted from the nozzle and contact the cooler environment. The result is a dry particle having an approximate eliptical or spherical shape. The particles are then screened to the desired size, usually to about 30 to about 200 U.S. standard mesh (about 600 to about 75 microns). The final product is a fine dry powder referred to herein as the delivery system. The sweetener is thus adequately coated and protected by the fat/rosin matrix and can be stored for later use, or directly incorporated into a chewing gum, confectionery, drug product, or personal product.

The process of preparing the inventive chewing gum compositons is as follows. The gum base is melted (about 85° to about 90°), cooled to 78° C and placed in a pre-warmed (60° C) standard mixing kettle equipped with sigma blades. The emulsifier ia then added. Next, a portion of the sorbitol and the glycerin is added and mixed for an additional 3 to 6 minutes. The mixing kettle is cooled and mannitol and the remainder of the sorbitol and glycerin are then added and mixing is continued. At this time, the unflavored chewing gum temperature is about 46°-51° C. Flavor oil is then added and incorporated into the base and the mixing is continued. Finally, the delivery system containing the encapsulated sweetener material is added and mixed for an additional 1 to 10 minutes. The delivery system is added as the last ingredient. The final gum temperature is about 48°C-54°C. The chewing gum composition is then discharged from the kettle, extruded, rolled, scored and formed into chewing gum pieces.

For Composition A (prior art) in Table I, the temperatures required to process the chewing gum composition of that delivery system were substantially lower than the temperatures used to process the inventive delivery systems. For example, the temperature of the unflavored chewing gum was required to be 39°-42°C and the final temperature of the gum was in the range of 39°-43°C. These lower temperatures were necessary to prevent this prior art delivery system from prematurely melting and releasing the sweetener during processing.

The chewing gum composition of this invention may additionally include the conventional additives such as emulsifiers, for example, lecithin and glyceryl monostearate; and fillers such as dicalcium phosphate, aluminum hydroxide, alumina, aluminum silicates, talc, calcium carbonate, and combinations thereof. The total amount of fillers present in generally up to about 10% by weight. Plasticizers or softeners such as lanolin stearic acid, sodium stearate as well as other conventional additives may be included to enhance the desired properties of the final chewing gum composition.

The inventive compositions can be used to prepare sugar or sugarless chewing gum compositions. Substantially anhydrous chewing gum compositions are contemplated as well.

The chewing gums of the invention may be in any form known in the art, such as stick gum, slab gum, chunk gum, shredded gum, hard coated gum, tableted gum, as well as center-filled gum.

The following examples are given to illustrate the invention, but are not deemed to be limiting thereof. All percentages given throughout the specification are based upon weight of the total chewing gum composition unless otherwise indicated.

## Examples

Table I sets forth delivery systems of the present invention (E,F,G,H,J,K) as well as those from Example One of U.S. Patent 4,597,970 (A).

Additionally, compositions B,C,D and I represent examples where only fat was used to encapsulate the sweetener. Attempts to uniformly disperse the sweetener using only fat failed, resulting in lumps of sweetener within the fat. Additionally, the viscosity of the mixture was too high to pump through the spray congealing nozzle. Attempts to process the fat sweetener mixture therefore failed. Hand mixing, pouring and grinding were the only means of producing a delivery system using just fat as the encapsulating matrix. Thus, although the melting points were within the range of Applicants' inventive delivery systems, these comparative compositions (B, C, D and I) are not operable in the instant invention because they cannot be easily spray congealed or otherwise made in a commercially acceptable and efficient manufacturing process. Conventional mixing or hand mixing is contrary to the state-of-the-art efficiency method of spray congealing. Additionally, as disclosed herein, spray congealing is the only method of effectively coating sweeteners such as aspartame, which due to their geometric configuration and sharp corners, are extremely difficult to properly encapsulate.

The melting point of each delivery system composition was taken using differential scanning colorimetry. The inventive delivery systems exhibited higher melting points (61.8° -67.7°C) than prior art composition A (56°C) and were easily processed into discrete particles, unlike those using pure fat as the encapsulating matrix.

The delivery systems were then incorporated into chewing gum compositions set forth in Table II and processed into final chewing gum product.

Each of the chewing gums were then tested for sweetness retention after processing at temperatures of about 48°C to about 54°C. The delivery system of composition A melted during the processing of the gum and was judged by an expert panel as having lost much of its sweetness.

After accelerated aging conditions, the loss of sweetness was exacerbated, indicating degradation of the encapsulant. The inventive delivery systems, on the other hand, retained full sweetness capacity due to the intactness of the fat/rosin encapsulation.

Table I

| DELIVERY SYSTEMS | | | | | | | |
|---|---|---|---|---|---|---|---|
| INGREDIENT | COMPOSITION - % WT. | | | | | | |
| | A | B | C | D | E | F | G |
| Sweetener* | 16.67 | 16.67 | 24.67 | 32.67 | 16.67 | 16.67 | 16.67 |
| Monoglyceride | 8.3 | - | - | - | - | - | - |
| Lecithin | 8.3 | - | - | - | - | - | - |
| Palm Oil | 67.73 | - | - | - | - | - | - |
| Cottonseed Oil | - | - | - | - | - | - | - |
| Soybean Oil | - | 83.33 | 75.33 | 67.33 | 78.33 | 73.33 | 68.33 |
| Wood Rosin Ester | - | - | - | - | 5 | 10 | 15 |
| Melting Point °C | 56 | 63.2 | 62.6/67.7 | 64.2/67.5 | 61.2 | 62.2 | 61.2/65.7 |
| | H | I | J | K | | | |
| Sweetener | 16.67 | 16.67 | 16.67 | 16.67 | | | |
| Monoglyceride | - | - | - | - | | | |
| Lecithin | - | - | - | - | | | |
| Palm Oil | - | - | - | - | | | |
| Cottonseed Oil | - | - | 78.33 | 73.33 | | | |
| Soybean Oil | 63.33 | 77.33 | - | - | | | |
| Wood Rosin Ester | 20 | - | 5 | 10 | | | |
| Melting Point °C | ** | 67.7 | 61.8 | 62.2 | | | |

*Aspartame
**Phase separation. No defined melting point.

TABLE II

| CHEWING GUM COMPOSITIONS | | | | | |
|---|---|---|---|---|---|
| INGREDIENT | COMPOSITION - % WT. | | | | |
| | PRIOR ART | | INVENTIVE | | |
| Delivery System | I | II | III | IV | V |
| A | 2.3 | - | - | - | - |
| B | - | 2.3 | - | - | - |
| E | - | - | 2.3 | - | - |
| F | - | - | - | 2.3 | - |
| G | - | - | - | - | 2.3 |
| gum base | 23.0 | 23.0 | 23.0 | 23.0 | 23.0 |
| sugar alcohol | 56.9 | 56.9 | 56.9 | 56.9 | 56.9 |
| *softener | 13.0 | 13.0 | 13.0 | 13.0 | 13.0 |
| emulsifier | 0.5 | 0.5 | 0.5 | 0.5 | 0.5 |
| flavor | 1.3 | 1.3 | 1.3 | 1.3 | 1.3 |
| color | 0.1 | 0.1 | 0.1 | 0.1 | 0.1 |
| filler | 2.9 | 2.9 | 2.9 | 2.9 | 2.9 |

*glycerin

The invention being thus described, it will be obvious that the same may be varied in many ways. Such variations are not to be regarded as a departure from the spirit and scope of the invention and all such modifications are intended to be included within the scope of the claims.

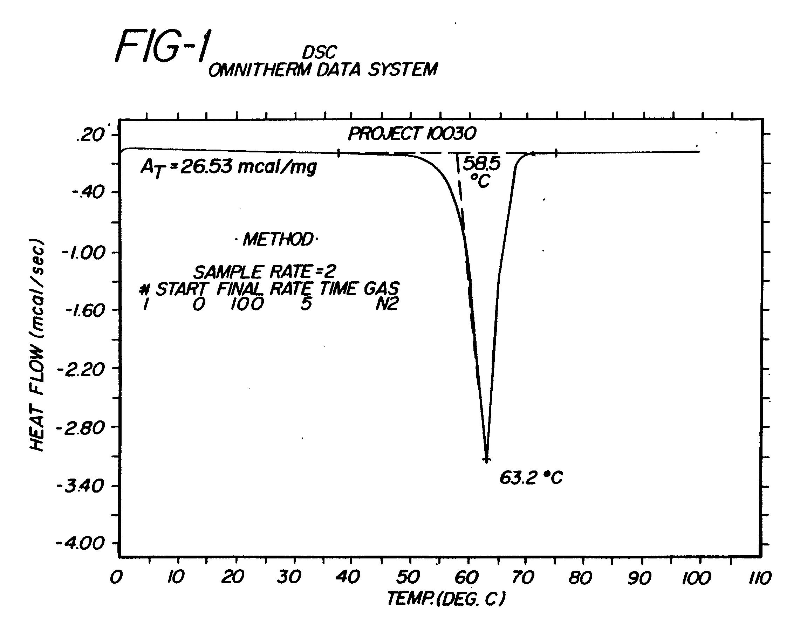

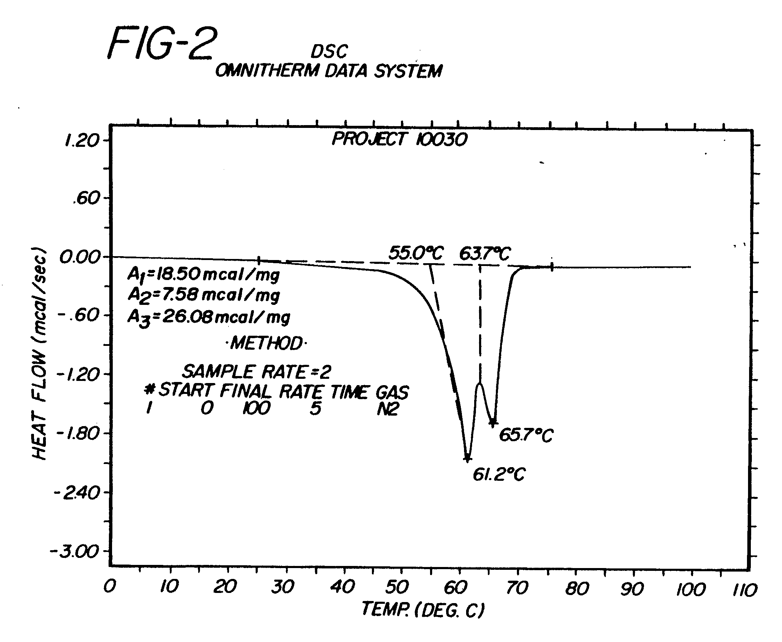

**Claims**

1. A high melting point ingestible sweetener delivery system, comprising a particulate sweetener encapsulated by a matrix comprising a blend of rosin and fat, wherein the rosin is selected from hydrogenated and partially hydrogenated wood or gum rosin esters and the fat is selected from hydrogenated and partially hydrogenated soybean oil, cottonseed oil and mixtures hereof.

2. A system according to claim 1, having a melting point range of about 60° to about 70°C.

3. A system according to claim 1 or 2, wherein the sweetener is selected from water-soluble natural sweeteners, water-soluble artificial sweeteners, dipeptide sweeteners, amino acid-based sweeteners and mixtures thereof.

4. A chewing gum composition having improved sustained sweetener release, comprising a gum base, a bulk sweetener and a high melting-point delivery system capable of withstanding a gum processing temperature of about 48° to about 54°C without melting or prematurely rupturing, said delivery system comprising a particulate sweetener and an encapsulating matrix comprising a blend of materials selected from hydrogenated wood rosin ester, partially hydrogenated wood rosin ester, hydrogenated gum rosin ester, partially hydrogenated gum rosin ester, soybean oil, cottonseed oil and mixtures thereof.

5. A composition according to claim 4, wherein the gum base is selected from synthetic gums, natural gums, synthetic elastomers, natural elastomers and mixtures thereof.

6. A composition according to claim 4 or 5, wherein the delivery system is present in an amount of about 0.1% to about 5% by weight.

7. A composition according to any of claims 4 to 6, wherein the bulk sweetener is selected from sucrose, glucose (corn syrup), dextrose, fructose, invert sugar, mannitol, xylitol, sorbitol and mixtures thereof.

8. A composition according to any of claims 4 to 7, wherein the encapsulating matrix has a melting point of about 60°C to about 70°C.

9. A composition according to any of claims 4 to 8, wherein the particulate sweetener in the delivery system is selected from saccharine and its salts, amino acid-based sweeteners, dipeptide sweeteners, dihydrochalcone compounds, talin, monellin, aspartame, glycyrrhizin, stevioside, sorbitol, mannitol, xylitol, hydrogenated starch hydrolysate, acesulfame and its salts, chlorinated sucrose derivatives and mixtures thereof.

10. A composition according to any of claims 4 to 9, wherein there is additionally present a material selected from fillers, plasticizers, emulsifiers, elastomer solvent, flavour, colour and mixtures thereof.

11. A composition according to claim 10, wherein the flavour is selected from natural flavours, artificial flavours and mixtures thereof.

Claims for the following Contracting State: ES

1. A process for preparing a high melting point ingestible sweetener delivery system, which comprises encapsulating a particulate sweetener on a matrix comprising a blend of rosin and fat, wherein the rosin is selected from hydrogenated and partially hydrogenated wood or gum rosin esters and the fat is selected from hydrogenated and partially hydrogenated soybean oil, cottonseed oil and mixtures hereof.

2. A process according to claim 1, wherein the system has a melting point range of about 60° to about 70°C.

3. A process according to claim 1 or 2, wherein the sweetener is selected from water-soluble natural sweeteners, water-soluble artificial sweeteners, dipeptide sweeteners, amino acid-based sweeteners and mixtures thereof.

4. A process for preparing a chewing gum composition having improved sustained sweetener release, which process comprises preparing the composition from a gum base, a bulk sweetener and a high melting-point delivery system capable of withstanding a gum processing temperature of about 48° to about 54°C without melting or prematurely rupturing, said delivery system comprising a particulate sweetener and an encapsulating matrix comprising a blend of materials selected from hydrogenated wood rosin ester, partially hydrogenated wood rosin ester, hydrogenated gum rosin ester, partially hydrogenated gum rosin ester, soybean oil, cottonseed oil and mixtures thereof.

5. A process according to claim 4, wherein the gum base is selected from synthetic gums, natural gums, synthetic elastomers, natural elastomers and mixtures thereof.

8

6. A process according to claim 4 or 5, wherein the delivery system is present in an amount of about 0.1% to about 5% by weight.

7. A process according to any of claims 4 to 6, wherein the bulk sweetener is selected from sucrose, glucose (corn syrup), dextrose, fructose, invert sugar, mannitol, xylitol, sorbitol and mixtures thereof.

8. A process according to any of claims 4 to 7, wherein the encapsulating matrix has a melting point of about 60°C to about 70°C.

9. A process according to any of claims 4 to 8, wherein the particulate sweetener in the delivery system is selected from saccharine and its salts, amino acid-based sweeteners, dipeptide sweeteners, dihydrochalcone compounds, talin, monellin, aspartame, glycyrrhizin, stevioside, sorbitol, mannitol, xylitol, hydrogenated starch hydrolysate, acesulfame and its salts, chlorinated sucrose derivatives and mixtures thereof.

10. A process according to any of claims 4 to 9, wherein there is additionally used a material selected from fillers, plasticizers, emulsifiers, elastomer solvent, flavour, colour and mixtures thereof.

11. A process according to claim 10, wherein the flavour is selected from natural flavours, artificial flavours and mixtures thereof.

FIG-1
DSC
OMNITHERM DATA SYSTEM

FIG-2
DSC OMNITHERM DATA SYSTEM